# EUROPÄISCHE PATENTSCHRIFT

(11) **EP 1 481 094 B1**
(45) Veröffentlichungstag und Bekanntmachung des Hinweises auf die Patenterteilung: **30.09.2009**
(21) Anmeldenummer: 03742959.4
(22) Anmeldetag: 26.02.2003
(51) Int. Cl.: C12Q 1/68

(54) **ERHÖHUNG DER SENSITIVITÄT UND SPEZIFITÄT VON HYBRIDISIERUNGSEXPERIMENTEN MIT NUKLEINSÄURE-CHIPS**
INCREASING THE SENSITIVITY AND SPECIFICITY OF NUCLEIC ACID CHIP HYBRIDIZATION TESTS
AUGMENTATION DE LA SENSIBILITE ET DE LA SPECIFICITE DE TESTS D'HYBRIDATION AVEC DES PUCES A ACIDE NUCLEIQUE

(30) Priorität: 28.02.2002 DE 10208770
(43) Veröffentlichungstag der Anmeldung: 01.12.2004
(73) Patentinhaber: febit holding GmbH, 69120 Heidelberg (DE)
(72) Erfinder: STÄHLER, Cord, F., 69469 Weinheim (DE); STÄHLER, Peer, F., 68167 Mannheim (DE); BEIER, Markus, 69121 Heidelberg (DE)
(74) Vertreter: Weiss, Wolfgang
(86) Internationale Anmeldenummer: PCT/EP2003/001972
(87) Internationale Veröffentlichungsnummer: WO 2003/072817

(56) Entgegenhaltungen:
- EP-A- 0 874 242
- WO-A-00/12123
- WO-A-02/40998
- WO-A-95/11995
- WO-A-98/08083
- US-B1- 6 255 677
- ZHIGUANG Y ET AL: "Multi-well ELISA based on independent peptide antigens for antibody capture Application to Lyme disease serodiagnosis" JOURNAL OF IMMUNOLOGICAL METHODS, ELSEVIER SCIENCE PUBLISHERS B.V.,AMSTERDAM, NL, Bd. 198, Nr. 1, 1996, Seiten 25-33, XP004021307 ISSN: 0022-1759
- LIPSHUTZ R J ET AL: "HIGH DENSITY SYNTHETIC OLIGONUCLEOTIDE ARRAYS" NATURE GENETICS, NEW YORK, NY, US, Bd. 21, Nr. SUPPL, Januar 1999 (1999-01), Seiten 20-24, XP000865982 ISSN: 1061-4036

## Beschreibung

Die Erfindung betrifft ein Verfahren zur Erhöhung der Sensitivität und Spezifität von Hybridisierungsexperimenten mit Nukleinsäure-Chips und zur Durchführung des Verfahrens geeignete Vorrichtungen.

DNA-Hybridisierung basiert auf der sequenzspezifischen Ausbildung eines komplementären Doppelstrangs aus unterschiedlichen Einzelstrangquellen unter bestimmten experimentellen Bedingungen. Kennt man eine Einzelstrangsequenz und benutzt diese als Sonde (z.B. in Form eines Oligonukleotids), kann nach Detektion eines Hybridisierungsereignisses, z.B. über Farbstoffmarkierung, die Zielsequenz abgeleitet werden. Dieser Prozess ist reversibel und kann über Temperaturänderungen gesteuert werden. Man macht sich diese Eigenschaft der DNA in verschiedenen Anwendungen zu Nutze, z.B. bei der Entschlüsselung der DNA-Sequenz (Sequencing by hybridisation, SbH) oder der Aktivitätsmessung unterschiedlicher Zellen oder von unterschiedlichen Zuständen in Zellen (Expression Profiling oder Gene Expression Monitoring), indem man die Kopienzahl der DNA-Transkripte (mRNA), die zu einem definierten Zeitpunkt in einer Zelle vorliegen, ermittelt. Hierbei müssen Hybridisierungsereignisse von Einzelstrang-DNA und mRNA quantitativ ausgewertet werden. Eine weitere wichtige Anwendung, ein Spezialfall der Sequenzierung, ist die Mutationsanalyse individueller DNA-Einzelpositionen (Single Nuclear Polymorphisms, SNP), welche der molekularen Krankheitsaufklärung dienen.

Für praktische Anwendungen hat sich die sogenannte Nukleinsäure-Chip-Technologie als vielversprechendes Werkzeug für oben genannte Fragestellungen etabliert. Hierbei werden Hybridisierungssonden bekannter Sequenz, z.B. Oligonukleotide oder cDNA-Moleküle, an definierten Positionen auf Oberflächen eines Trägers immobilisiert und dienen dort als Fängermoleküle für Zielsequenzen aus unterschiedlichem Probenmaterial. Über den Nachweis der Hybridisierung von Zielsequenzen an die Oberflächen, z.B. durch Markierung des Probenmaterials mit Fluoreszenzfarbstoffen, ergeben sich in einem Hybridisierungsexperiment Messsignale, die mit Hilfe von geeigneten Methoden ausgewertet werden können. Aufgrund der Kenntnis der Sondensequenz können die Zielsequenzen im Probenmaterial identifiziert und charakterisiert werden.

Die Hybridisierung auf einer Festphase, z.B. DNA-Chip, DNA-Array oder DNA-Filter, ist ein diffusionsabhängiger Prozess, der von einem komplexen Zusammenwirken verschiedener Faktoren, u.a. von
a) der Reaktionstemperatur,
b) den Pufferbedingungen,
c) den relativen Einzelstrangkonzentrationen,
d) der Weglänge zwischen Probenmolekül und Sonde sowie
e) der Zahl der DNA-DNA-Wechselwirkungen, die auf diesem Weg stattfinden,
abhängt. In der praktischen Anwendung, z.B. bei Expression Profiling, SbH u.a., stößt man aufgrund der Komplexität der DNA-Moleküle im Probenmaterial, der üblicherweise großen Probenvolumina und Chipflächen auf starke Limitationen hinsichtlich:
a) der Genauigkeit bzw. der Spezifität (Qualität),
b) der Empfindlichkeit (Probenmenge, Komplexität des Probenmaterials)
c) des Durchsatzes (Geschwindigkeit, Kosten) und
d) der möglichen Ausführungsformen (Nutzen für Forschung und Diagnostik).

Die Nukleinsäure-Hybridisierung ist ein Gleichgewichtsprozess, der nach dem Massenwirkungsgesetz beschrieben werden kann: [A] (Sonde) + [B] (Zielsequenz) ↔ [AB]. Da [A], d.h. die Konzentration der Sonde, die auf einem Chip immobilisiert ist für alle immobilisierten Sonden (A1 bis An) nach dem Stand der Technik üblicherweise annähernd konstant ist, ergeben sich für die relative Quantifizierung der Zielsequenzen (B) in Probengemischen (B1 bis Bn), Probleme, wenn [B1] bis [Bn] (d.h., die Konzentration der einzelnen Zielsequenzen B1 bis Bn) nicht konstant ist. Dies ist z.B. bei der Genexpressionsprofilierung der Fall. Individuelle Zielsequenzkonzentrationen können um einen Faktor von 10.000 schwanken. Das hat zur Folge, dass einige Sondenstellplätze auf dem Chip in einem Experiment gegenüber anderen Sondenstellplätzen physikalisch gesättigt werden können und bei der Detektion der lineare dynamische Messbereich überschritten wird, wodurch eine Quantifizierung aller Signale in einer einzigen Messung nicht möglich ist. Dies beeinflusst Sensitivität (Qualität) und Anwendungsmöglichkeiten (Nutzen).

Zum Beispiel besteht bei der Anwendung SbH das Problem, dass im Fall eukaryontischer Zielsequenzen ein Großteil an repetitiven Sequenzen (beim humanen Genom: 97 %) vorliegt, wodurch die relative Konzentration des relevanten Zielsequenzbereichs fast um den Faktor 100 niedriger ist, als sie es sein könnte, wenn man z.B. die repetitiven Sequenzen vor dem Experiment "herausfiltern" würde. Dies beeinflusst Sensitivität und Spezifität (Qualität) und behindert viele denkbare Anwendungen mit DNA-Chips.

Ein weiteres Problem bei der Anwendung SbH besteht darin, dass es prinzipiell nicht möglich ist, bei einer definierten Hybridisierungstemperatur alle beliebigen DNA-Doppelstränge spezifisch auszubilden (Qualität, Nutzen), da die DNA-Hybridisierung kinetisch kontrolliert ist und sich Doppelstränge ausbilden, die nicht dem thermodynamischen Minimum entsprechen. Erst durch reversible Auflösung unspezifisch gebundener DNA-Moleküle und durch Einstellen der individuellen Duplexschmelztemperatur kann die Reaktion in Richtung des thermodynamisch günstigsten Zustandes ermöglicht werden (spezifische Doppelstrangausbildung).

Untersuchungen zur Bestimmung von Analyten auf Festphasen sind bekannt, wie z.B. von R. P. Ekins, US-Patent 5 432 099, beschrieben. Die bekannten DNA-Chip-Hybridisierungsverfahren können in zwei Kategorien eingeteilt werden: Das passive Hybridierungsverfahren und das aktiv unterstützte Hybridisierungsverfahren.

Bei der passiven Hybridisierung ist die Probenlösung stationär und es wird bei einer definierten Temperatur gearbeitet, wobei die Hybridisierung diffusionsabhängig erfolgt. Zu dieser Kategorie gehören die flächige Slide- oder Array-Technologie unter Verwendung von Chips, die durch Spotting oder in situ-Synthese hergestellt werden. Der Vorteil dieser Technologien ist, dass sie eine relativ hohe Stellplatzdichte aufweisen. Nachteilig erweist sich jedoch, dass man große Probenvolumina gebrauchen muss, dass nur eine niedrige lokale Zielsequenzkonzentration entsteht, die u.a. eine sehr langsame Hybridisierung (ca. 16-48 h) verursacht und dass der nutzbare lineare Messbereich nur 2 - 3 Zehnerpotenzen überstreicht. Ein weiterer Nachteil entsteht durch die flächige einheitliche Temperatur, die zu unspezifischen (falsch-positiven) Hybridisierungsergebnissen führen kann. Bei SbH mit repetitiver DNA ist bei dieser Technologie das Signal-Hintergrund-Verhältnis niedrig bis nicht messbar.

Bei den aktiv unterstützten Hybridisierungverfahren wird die Probenlösung durch Kanäle oder mit Hilfe elektrischer Felder über die immobilisierten Sonden bewegt und es kann ein Temperaturgradient eingestellt werden. Zu dieser Kategorie gehört die 3-D-Chip-Technologie mit Kanalgeometrie. Vorteilhaft ist bei dieser Technologie, dass die Hybridisierungszeiten durch die aktive Bewegung der Probe kurz sind und dass man relativ kleine Probenvolumina benutzen kann. Nachteilig erweist sich jedoch die niedrige Stellplatzdichte und dass Keine lokale Temperaturkontrolle eingestellt werden kann, was zu falsch-positiven Ereignissen führen kann.

Ein weiteres aktiv unterstütztes Hybridisierungsverfahren ist die "96-well printing"-Technologie in Mikrotiterplatten. Diese Technologie hat zum Vorteil, dass die einzelnen Mikrotiterplatten-Vertiefungen individuell temperatursteuerbar sind, z.B. mit Hilfe einer PCR-Maschine. Nachteilig ist dennoch der Gebrauch von sehr großen Probenvolumina, die niedrige Stellplatzdichte und die diffusionsabhängige und langsame Hybridisierung, die die Sensitivität dieses Verfahrens sehr beeinflusst.

Zuletzt ist die elektronisch gesteuerte Hybridisierung bekannt. In dieser Art der aktiv unterstützten Hybridisierung wird die Probe zur Sonde bewegt. Das bewirkt, dass man sehr kurze Hybridisierungszeiten hat. Durch die Elektronik entsteht eine temperatur-äquivalente Stringenz und es ermöglicht eine Falschpositiven-Diskriminierung. Trotzdem ist es eine recht teure Technologie mit sehr niedrigen Integrationsdichten, die nicht ohne weiteres einsetzbar ist für SbH und genomisches Expression Profiling.

WO 95/11995 betrifft Arrays aus Oligonukleotidsonden, welche in mikrogefertigten Mustern auf Siliciumchips immobilisiert sind, um molekulare Wechselwirkungen zu untersuchen.

Yu et al. (J. Immunol. Methods (1996), 198 25-33) beschreiben Verfahren zur Verwendung von Peptiden als Reagenzien zum Abfangen von Antikörpern im ELISA-Testformat.

Lipshutz et al. (Nature Genetics Supplements (1999), 21 20-24) offenbaren zweidimensionale Arrays synthetischer Oligonukleotide, die zur quantitativen und parallelen Messung der Expression von Genen konzipiert sind.

US 6,255,677 ist auf eine Analysevorrichtung, umfassend einen mit einer Vielzahl von Elektroden ausgestatteten elektronischen Chip, gerichtet. Die auf dem Chip positionierten Elektroden können individuell mit einzelnen Verbindungen beschichtet werden, welche eine Spezifität für bestimmte chemische und biologische Produkte besitzen.

WO 98/08083 betrifft Verfahren sowie Systeme zur molekularen Erkennung, wobei eine zu untersuchende Probe an Bindungsstellen einer geeigneten Vorrichtung gebunden und das erhaltene Muster zur Bestimmung der Molekülstruktur mit einem Referenzmuster verglichen wird.

WO 00/12123 beschreibt ein Verfahren zur Bestimmung einer Vielzahl von Analyten in einer Probe sowie eine zur Durchführung des Verfahrens geeignete Messvorrichtung.

EP 0 874 242 A1 offenbart eine Vorrichtung zur gleichzeitigen Detektion einer Vielzahl von Analyten, umfassend einen Träger sowie eine Vielzahl einzelner Reaktionsbereiche, in welchen jeweils ein Ligand kovalent an den Träger gebunden ist, wobei die Oberfläche des Trägers zwischen den einzelnen Reaktionsbereichen in Bezug auf den Analyten inert ist.

Zusammenfassend lässt sich sagen, dass bisher etablierte Verfahren zum Nachweis von Nukleinsäuren durch Hybridisierung unter Verwendung von Chips mit immobilisierten Hybridisierungssonden der Komplexität von biologischem Probenmaterial nicht angemessen angepasst werden können. Eine Aufgabe der vorliegenden Erfindung war es daher Verfahren und Systeme zur Bestimmung von Analyten auf Trägern, z. B. Chips, bereitzustellen, bei denen die Nachteile des Standes der Technik mindestens teilweise vermieden werden.

Diese Aufgabe wird durch ein Verfahren gelöst, bei dem einzelne Bereiche oder Gruppen von Bereichen von Hybridisierungssonden auf dem Träger variabel für die jeweils gewünschte Anwendung ausgestaltet sind, so dass die Sensitivität, die Spezifität und die Wirtschaftlichkeit erheblich verbessert werden. Das erfindungsgemäße Verfahren ermöglicht die Bindung der Analyten an eine Sonde durch Hybridisierung.

Gegenstand der vorliegenden Erfindung ist somit ein 5.6a.

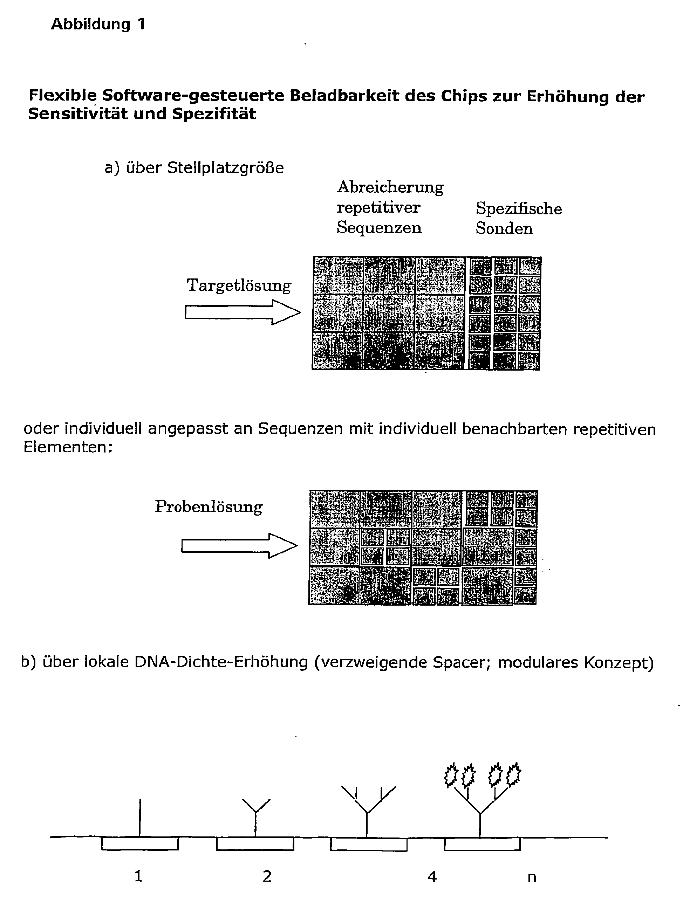

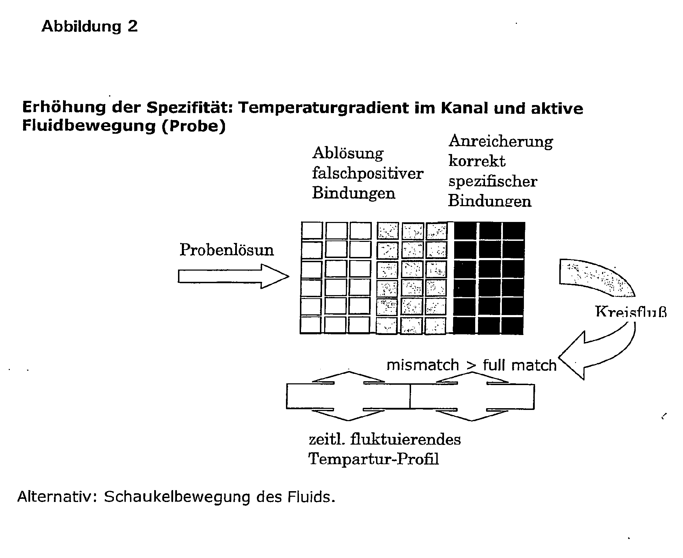

Vorzugsweise basiert das erfindungsgemäße Verfahren auf der Geniom^{®}-Technologie, die in WO 00/13018 beschrieben ist. Es kann die geometrischen Strukturen (Mikrokanäle), die flexible Belastbarkeit des Fluidprozessors (d.h., die unterschiedlichen lokalen Rezeptorkonzentrationen, wie in Abb. 1 dargestellt), und die Möglichkeit der aktiven Fluidbewegung in Kombination mit der Möglichkeit zur lokalen Temperaturführung (wie in Abb. 2 dargestellt) nutzen. Verfahren zur Bestimmung von Nukleinsäureanalyten durch Hybridisierung, umfassend die Schritte
(a) Bereitstellen eines mikrofluidischen Trägers mit Kanälen,
(b) Synthetisieren von jeweils unterschiedlichen Rezeptoren in situ an mehreren vorbestimmten Bereichen auf dem mikrofluidischen Träger, wobei die Rezeptoren an den mehreren vorbestimmten Bereichen auf dem Träger immobilisiert werden und sich die mehreren vorbestimmten Bereiche mit immobilisierten Rezeptoren in den Kanälen des mikrofluidischen Trägers befinden,
(c) Inkontaktbringen des mikrofluidischen Trägers mit einer Analyten enthaltenden Probe und
(d) Bestimmen der Analyten über deren Bindung an die auf dem mikrofluidischen Träger immobilisierten Rezeptoren,
wobei für vorbestimmte Bereiche oder Gruppen von Bereichen mit Rezeptoren jeweils eine unterschiedliche Intensität des Messsignals durch lokal unterschiedliche Einstrahlung von Anregungslicht zur Anpassung der Fluoreszenzintensität in den vorbestimmten Bereichen erzeugt wird so dass der lineare dynamische Messbereich des Detektors nicht überschritten wird, und der Nachweis des Analyten durch Fluoreszenz erfolgt, und wobei die Rezeptoren Hybridisierungssonden sind.

Insbesondere die mit der Geniom^{®}-Technologie besonders kurzen Produktionszeiten neuer DNA-Chips (innerhalb weniger Stunden) und die dadurch ermöglichten kurzen Lernzyklen machen diese Applikationen mit DNA-Chips technisch nicht nur durchführbar, sondern auch wirtschaftlich, wie z.B. Sequenzierung (SbH) von DNA mit hohem repetitiven DNA-Anteil, Expression Profiling mit ausreichendem dynamischen Sensitivitätsbereich, damit in komplexen mRNA-Proben sowohl sehr seltene als auch sehr häufige Transkripte quantitativ erfasst werden können, und massiv parallele SNP-Detektion mit hoher individueller Oligo-Duplexspezifität.

Im erfindungsgemäßen Verfahren können vorzugsweise durch Verwendung der Geniom^{®}-Technologie, z.B. unter Verwendung eines integrierten Synthese- und Analysesystems (ISA-System), sowohl bei der Vorbereitung eines Tests als auch während eines Tests (Online-Detektion) oder/und bei der Auswertung eines Tests (Lernendes System) biophysikalische Parameter, wie zu vor angegeben variiert werden. Diese Manipulation der Hybridisierungssignal beeinflussenden Parameter können sowohl global, als auch lokal (also für jeden Oligonukleotidsensor individuell) erfolgen.

Eine weitere Verbesserung, kann beispielsweise bei der Expression Profiling Anwendung, erreicht werden. Konstitutiv hoch-exprimierte Gensequenzen können über Flächen, die bis zu 100x größer sind als die anderen und bis zu 10x höhere Stellplatzdichten haben, abgereichert werden. Dadurch wird die Sensitivität für selten exprimierte Gene erhöht. Diese Sensitivität kann durch Lernzyklen optimiert werden, wobei für die bei einem ersten Experiment identifizierten Gene anhand der relativen Häufigkeit (Fluoreszenzintensität) ein neuer Chip programmiert wird, der Unterschiede über die Größe oder/und Rezeptordichte der Stellplätze ausgleicht, so dass ein möglichst homogenes Messsignal entsteht. Auch mit unterschiedlicher Belichtungszeit (-mengen) pro Stellplatz unter Verwendung einer Lichtquellenmatrix kann die Sensitivität optimiert werden. Mit dieser Belichtungseinstellung kann nun Testmaterial untersucht werden. Das System wird sensitiver und der Dynamikbereich wird in den linearen Bereich verschoben.

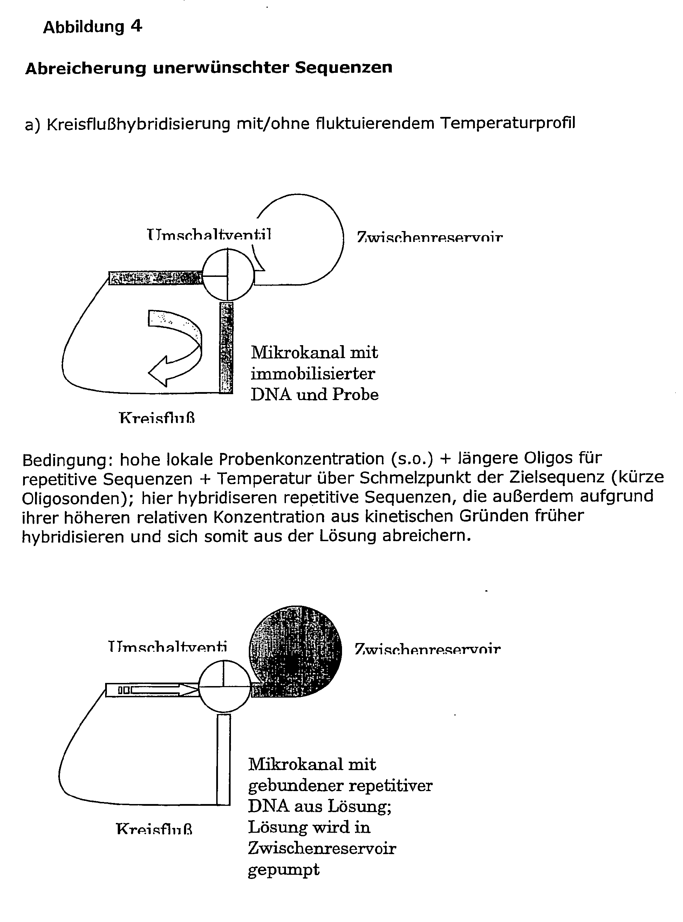

Auch in der SbH-Anwendung kann eine zusätzliche Verbesserung erreicht werden. Repetitive DNA kann in Regionen des Chips abgereichert werden, indem, auf geeignet großen Flächen, spezielle Spacer immobilisiert werden, die eine höhere 3-dimensionale Verzweigung und eine größere lokale Stellplatzdichte aufweisen und so die repetitive Sequenzen "herausfiltern". Dadurch wird die eigentliche Messung sensitiver und liefert ein besseres Signal-Hintergrund-Verhältnis. Dieser Effekt kann durch sehr schnelle Reassoziationskinetik beschleunigt werden: eine Hybridisierung wird in einer Minute durchgeführt, so dass häufig vorkommende Sequenzen schnell zu ihrer Sonde finden. Anschließend wird die Lösung abgenommen und in ein Reservoir zwischengelagert (siehe Abb. 4). Die hybridisierte DNA wird mit heißer Lösung abgelöst und entfernt. Dieser Zyklus wird wiederholt bis nach mehreren solcher Zyklen, die Hybridisierungslösung in den Messkanal oder eventuell in den gleichen Kanal eingeführt werden kann. Je nach Experiment können mit Hilfe der variablen Stellplatzdichte, der aktiven Fluidbewegung und einer lokal unterschiedlichen Temperierung gezielt spezifische repetitive Sequenzen abgereicht werden.

Zusätzlich kann man mehrere überlappende oder nichtüberlappende Rezeptoren für ein Gen in Nachbarschaft zueinander anordnen, um über unterschiedlich große Flächen individueller Rezeptoren den Massenwirkungseffekt auszunutzen, um Affinitätsunterschiede auszugleichen, d.h. die lokale Konzentration einer Oligonukleotidsonde wird variiert. Dies bedeutet, dass die Unterschiede in der Schmelztemperatur verschiedener Oligonukleotide, z.B. durch individuelle Anpassung der Stellplatzgröße, ausgeglichen werden. So wird einer Oligonukleotidsonde mit geringerer Schmelztemperatur, z.B. verursacht durch hohen AT-Gehalt, eine entsprechend größere Stellplatzfläche zugewiesen als einer Sonde mit höherem Schmelzpunkt, z.B. verursacht durch höheren GC-Gehalt. Bei einer späteren Signalquantifizierung können die größeren Stellplatzflächen integriert und wie ein Standard-Signal gewertet werden (Lernprinzip).

Eine Anpassung unterschiedlicher Schmelzpunkte von Rezeptorsonden kann neben der Veränderung der Fläche auch durch die Variation der Flächendichte erfolgen. Dies wird z.B. dadurch bewerkstelligt, dass die lokale Rezeptordichte über verzweigte (dendrimere) Strukturen (vgl. Abb. 1b) eingestellt wird. Einer Sonde mit tiefem Schmelzpunkt wird z. B. eine verzweigte Struktur mit hohem Verzweigungsgrad zugeordnet und entsprechend einer Sonde mit hoher Schmelztemperatur eine mit entsprechend niedrigem Verzweigungsgrad.

In einer ersten Ausführungsform des Verfahrens sind ein oder mehrere vorbestimmte Bereiche mit Rezeptoren unterschiedlich ausgestaltet, d.h. es werden unterschiedliche Bedingungen für die lokale Rezeptorkonzentration aus unterschiedlichen Bereichsgrößen, d.h. Stellplatzgrößen für einzelne Rezeptoren oder/und unterschiedlichen Rezeptordichten innerhalb der Bereiche ausgewählt. Dabei weisen die Bereiche die zur Bindung von Molekülen die in der Probe häufig vorkommen, z.B. Bereiche die zur Bindung von repetitiven Sequenzen oder Bereiche die zur Bindung von konstitutiv hoch-exprimierte Gene dienen, eine erhöhte lokale Rezeptorkonzentration auf.

Unterschiedliche Stellplatzgrößen können während der Synthese der Rezeptoren durch unterschiedlich große Synthesefelder, z.B. durch Verwendung einer entsprechenden Software, realisiert werden. Vorzugsweise werden die Größen der einzelnen Bereiche um mindestens 50 %, besonders bevorzugt um mindestens 100 % (basierend auf der Größe des kleinsten Bereiches) variiert (siehe z.B. Abb. 1a).

Unterschiedliche Stellplatzdichten können über die Synthesechemie durch Verwendung unterschiedlicher Reagenzien, z.B. Spacern mit unterschiedlichen Verzweigungsgraden, realisiert werden (siehe z.B. Abb. 1b). Vorzugsweise werden die Rezeptordichten einzelner Bereiche um mindestens 50 %, besonders bevorzugt um mindestens 100 % (basierend auf dem Bereich mit der geringsten Rezeptordichte) variiert.

Bisherige Verfahren ermöglichen keine große Variationsmöglichkeiten, was die Mengen bzw. lokalen Konzentrationen der Sonden betrifft, so dass keine individuelle Anpassung an das stark variierende Probenmaterial getroffen werden kann. Durch die hier beschriebene beliebig große Variabilität in der Stellplatzfläche und sogar der lokalen Rezeptorkonzentration pro Stellplatz (Stellplatzdichte) kann mit Hilfe zweier Lernzyklen eine Anpassung an ein definiertes Probenmaterial erfolgen, um die Sensitivität der Messung zu optimieren.

Weiterhin können einzelne Bereiche oder Gruppen von Bereichen mit Rezeptoren unterschiedliche Bedingungen für die Rezeptor-Ligand-Affinität aufweisen. Dies wird durch unterschiedliche Rezeptorlängen oder/und unterschiedliche Arten der Rezeptorbausteine, z.B. PNA- oder LNA-Bausteine in den einzelnen Bereichen realisiert. Vorzugsweise wird die Rezeptorlänge einzelner Bereiche um mindestens 20 %, besonders bevorzugt um mindestens 50 % (basierend auf dem Bereich mit der kürzesten Rezeptorlänge) variiert.

In einer weiteren Ausführungsform werden in einem oder mehreren vorbestimmten Bereichen mit Rezeptoren unterschiedliche Bedingungen für die Kinetik der Rezeptor-Analyt-Wechselwirkung eingestellt, z.B. ausgewählt aus unterschiedlichen Temperaturen oder/und Temperaturprofilen in den Bereichen oder/und unterschiedlichen Fluidbedingungen in den Bereichen.

Die Temperatur kann über den gesamten Träger, z.B. gesamtflächig als stationärer oder fluktuierender Temperaturgradient oder/und lokal über einzelne Bereiche oder Gruppen von Bereichen, z.B. stellplatzspezifisch , variiert werden. Die gesamtflächige Temperatursteuerung kann mit Hilfe eines Peltier-Elements oder durch temperierte Luftströmung realisiert werden. Die lokale Temperatursteuerung kann durch das ortsspezifische Einstrahlen von Energie, z.B. als IR-Strahlung mit Hilfe einer Lichtquellenmatrix realisiert werden, wobei individuelle Stellplätze mit einer individuell eingestellten Lichtmenge beleuchtet werden, so dass durch Absorption Wärme entsteht. Die Einstrahlung ist hier proportional zur Wärmeentwicklung und zur Temperaturerhöhung. Alternativ oder zusätzlich kann die lokale Stellplatzflächentemperatur durch Elektronenströme in Leiterbahnen, die im Träger über einzelne Bereiche verlaufen, geregelt werden. Durch diese Temperaturkontrolle kann erfindungsgemäß auch in den einzelnen Bereichen oder Gruppen von Bereichen mit Rezeptoren ein fluktuierender Temperaturgradient eingestellt werden.

Unterschiedliche Doppelstränge, die durch Hybridisierung von Rezeptoren auf den Trägern und Zielsequenzen in der Probe entstehen, die in einem parallelen Verfahren auf einem einzigen Chip analysiert werden sollen, haben unterschiedliche Hybridisierungskinetiken und Schmelzkurven. Durch die hier beschriebenen fluktuierenden Temperaturgradienten und lokal individuell einstellbare Temperaturen, z.B. mit Hilfe einer Lichtquellenmatrix, wird dieses bisher "prinzipielle" Problem der Spezifität in parallelen Messungen gelöst.

Dabei werden vorzugsweise die Temperaturen in den einzelnen Bereichen um mindestens 2 °C, oftmals um mindestens 5 °C und in einigen Fällen um mindestens 10 °C variiert.

Eine weitere Möglichkeit, die Bedingungen für die Kinetik der Rezeptor-Analyt-Wechselwirkung in einzelnen Bereichen des Trägers zu variieren, ist das Einstellen unterschiedlicher Fluidbewegungen in einem oder mehreren unterschiedlichen Bereichen des Trägers. Dabei kann die Probe während des Hybridisierungsprozesses im Fluidprozessor z.B. mit Hilfe von Pumpen (Kolbenpumpen, Gasdruckpumpen) aktiv bewegt werden. Vorzugsweise wird die Probe im Kreisfluss oder/und in einer Schaukelbewegung aktiv über den Träger bewegt.

Vorzugsweise wird dabei die Fluidgeschwindigkeit in einzelnen Bereichen des Trägers um mindestens 20 %, vorzugsweise mindestens 50 % (basierend auf dem Bereich mit der geringsten Fluidgeschwindigkeit) variiert.

Durch aktive Fluidbewegung kann die Probe aktiv an der Sonde vorbeibewegt werden, wodurch einerseits die Hybridisierungsgeschwindigkeit erhöht und andererseits ein Trennprinzip genutzt werden kann, um unterschiedlich hybridisierende Probenelemente nach Hybridisierung voneinander zu trennen (chromatographisches Prinzip). In Kombination mit fluktuierenden Temperaturgradienten kann so Spezifität und Sensitivität erhöht werden. Demzufolge kann eine ein- oder mehrfache Rückführung der Probe, unter verschiedenen kinetischen Bedingungen, über den Träger erfolgen. Dabei kann pro Zyklus ein steigendes Temperaturprofil oder/und ein sinkendes Temperaturprofil oder/und eine Kombination aus steigendem und sinkendem Temperaturprofil eingestellt werden.

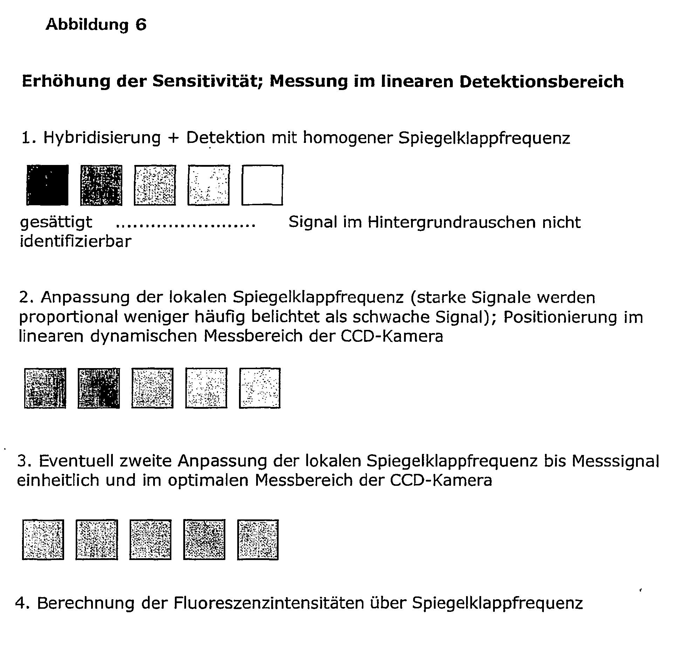

Ein Parameter, der in dem erfindungsgemäßen Verfahren variiert wird, ist die virtuelle Konzentration des Analyten. Hierzu werden unterschiedliche Bedingungen für das Bestimmen der Konzentration des Analyten erzeugt. Diese umfassen eine Erzeugung oder/und Detektion des Messsignals in einzelnen Bereichen mit unterschiedlicher Intensität. Der Nachweis des Analyten erfolgt durch Fluoreszenz und die unterschiedliche Intensität des Messsignals wird durch eine lokal unterschiedliche Einstrahlung von Anregungslicht, vorzugsweise über eine Lichtquellenmatrix, erzeugt, so dass der lineare dynamische Messbereich des Detektors nicht überschritten wird. Die individuelle Beleuchtungsintensität der Bereiche variiert erfindungsgemäß vorzugsweise um mindestens 50 %, besonders bevorzugt um mindestens 100 % (basierend auf dem Bereich mit der geringsten Beleuchtungsintensität). Die erfindungsgemäße lokal variable Belichtung über eine Lichtquellenmatrix wird in Abbildung 6 schematisch dargestellt.

Bisherige Verfahren ermöglichen keine steuerbare individuelle Belichtung einzelner Stellplätze zur Anpassung der Fluoreszenzintensitäten über die Anregungslichtmenge (unterschiedliche Belichtung individueller Stellplätze). Mit Hilfe der Lichtquellenmatrix kann nach einer ersten Testmessung eine individuelle Belichtung einzelner Stellplätze erfolgen, wodurch unterschiedliche Intensitäten der Fluoreszenzemission in einzelnen Bereichen ausgeglichen werden können, so dass der lineare dynamische Messbereich des Detektors, z.B. einer CCD-Kamera, nicht überschritten wird. Hierdurch steigt insbesondere die Sensitivität und die Genauigkeit quantitativer Messungen. Beispielsweise werden Stellplätze mit Rezeptoren, die geringere Schmelztemperaturen aufweisen, länger belichtet, solche mit höherem Schmelzpunkt kürzer, so dass die Signale beider Sonden eine vergleichbare Intensität aufweisen. Dies ist besonders auch für Anwendungen, die keine quantitative Auswertung erfordern, sondern auf einer ja/nein- Entscheidung beruhen, wichtig. Dies sind z.B. SNP-Analysen oder Resequencing-Applikationen, bei welchen bestimmte Zielsequenzen problembehaftet sind, d.h. für Hybridisierungen schwerer zugänglich sind, Respektive die entsprechenden Hybridisierungssignale niedrig ausfallen und es erforderlich ist, diese zu verstärken, was dann durch lokale längere Belichtungszeiten erfolgen kann.

Der Träger ist nach dem erfindungsgemäßen Verfahren eine Flusszelle bzw. eine Mikroflusszelle, d.h. ein mikrofluidischer Träger mit Kanälen, vorzugsweise mit geschlossenen Kanälen, in denen sich die vorbestimmten Positionen mit den jeweils unterschiedlichen immobilisierten Rezeptoren befinden. Die Kanäle haben vorzugsweise Durchmesser im Bereich von 10 bis 10 000 µm, besonders bevorzugt von 50 bis 250 µm und können grundsätzlich in beliebiger Form ausgestaltet sein, z.B. mit rundem, ovalem, quadratischem oder rechteckigem Querschnitt.

Die Rezeptoren werden ausgewählt aus Hybridisierungssonden, z.B. Nukleinsäuren, wie DNA und RNA, oder Nukleinsäureanaloga, wie Peptidnukleinsäuren (PNA), und Locked-Nukleinsäuren (LNA).

Die Bindung der Analyten umfasst eine Hybridisierung.

Das erfindungsgemäße Verfahren umfasst eine parallele Bestimmung von mehreren Analyten, d.h. es wird ein Träger bereitgestellt, der mehrere unterschiedliche Rezeptoren, die mit jeweils unterschiedlichen Analyten in einer einzigen Probe reagieren können, enthält. Vorzugsweise werden durch das erfindungsgemäße Verfahren mindestens 50, vorzugsweise mindestens 100 Analyten parallel in der Probe bestimmt.

Zur Durchführung des erfindungsgemäßen Verfahrens wird günstigerweise eine Vorrichtung verwendet, umfassend:
(i) eine Lichtquellenmatrix,
(ii) einen mikrofluidischen Träger mit mehreren vorbestimmten Positionen, an denen jeweils unterschiedliche Rezeptoren, ausgewählt aus Nukleinsäuren und Nukleinsäureanaloga, auf dem Träger immobilisiert sind,
(iii) Mittel zur Zufuhr von Fluids zum Träger und zur Ableitung von Fluids aus dem Träger und
(iv) eine Detektionsmatrix, umfassend mehrere Detektoren, die den vorbestimmten Bereichen auf dem Träger zugeordnet sind.

Eine derartige Vorrichtung ist eine aus den deutschen Patentanmeldungen 198 39 254.0, 199 07 080.6 und 199 40 799.5 bekannte Lichtemissions-Detektionseinrichtung, die in einem Gerät vereinigt ist, so dass damit das erfindungsgemäße Verfahren in Form einer zyklischen integrierten Synthese und Analyse durchführbar ist. In der Vorrichtung ist besonders bevorzugt, dass eine programmierbare Lichtquellenmatrix verwendet wird, ausgewählt aus einer Lichtventilmatrix, einem SpiegelArray und einem UV-Laser-Array. In der Vorrichtung können zwei Lichtquellenmatrices verwendet werden, wobei die eine zur Temperatursteuerung und die andere zur Detektion der Messsignale, im Falle, dass der Nachweis des Analyten durch Fluoreszenz erfolgt, dienen. Weiter ist es erfindungsgemäß bevorzugt, dass eine programmierbare Detektionsmatrix verwendet wird, ausgewählt aus einem CCD-Array, lichtsensitiven Halbleiterstrukturen und elektronischen Detektoren. Die Vorrichtung kann zur gesteuerten in situ Synthese der Rezeptoren benutzt werden. Die Synthese der Rezeptoren umfasst das Leiten von Fluid mit Rezeptor-Synthesebausteinen über den Träger, das orts- oder/und zeitspezifische Immobilisieren der Bausteine an den jeweils vorbestimmten Bereichen auf dem Träger und das Wiederholen dieser Schritte, bis die gewünschten Rezeptoren an den jeweils vorbestimmten Bereichen synthetisiert worden sind. Weiterhin umfasst die Rezeptorsynthese mindestens einen fluidchemischen Reaktionsschritt oder/und mindestens einen Belichtungsschritt oder/und einen elektrochemischen Reaktionsschritt oder/und eine Kombination solcher Schritte.

Weiterhin soll die vorliegende Erfindung durch die nachfolgenden Abbildungen erläutert werden:
In **Abbildung 1** wird die flexible Beladbarkeit der Chips schematisch dargestellt, die zu einer Erhöhung der Sensitivität und der Spezifität der Hybridisierungsexperimente dienen kann. Wie in Abbildung 1a dargestellt, werden unterschiedliche Stellplatzgrößen durch unterschiedlich große Synthesefelder realisiert, wobei große Flächen für die Abreicherung repetitiver und hochexprimierter Gensequenzen und kleine Flächen für die spezifischen Sonden realisiert werden. Abbildung 1b zeigt, wie durch unterschiedlich verzweigte Spacer die lokale Rezeptor-Dichte in den einzelnen Stellplätzen erhöht werden kann.
**Abbildung 2** zeigt wie man durch das Einstellen eines zeitlich fluktuierenden Temperaturgradienten auf dem Träger und durch eine aktive Fluidbewegung der Probe eine Erhöhung der Spezifität des Hybridisierungsverfahren erreichen kann. Durch das fluktuierende Temperaturprofil und durch die Fluidbewegung (Kreisfluss oder/und Schaukelbewegung des Fluids) erreicht man eine Ablösung der falschpositiven Bindungen und gleichzeitig eine Anreicherung der korrekten spezifischen Bindungen.

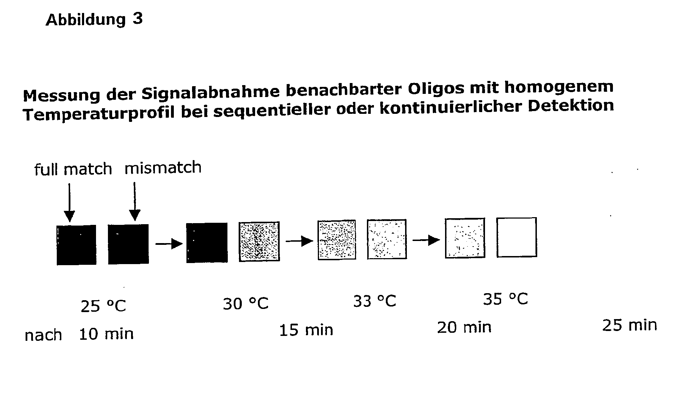

**Abbildung 3** zeigt die Messung der Signalabnahme benachbarter Rezeptoren mit homogenem langsam ansteigenden Temperaturprofil bei sequenzieller oder kontinuierlicher Detektion. Man kann erkennen, wie durch die Temperaturerhöhung eine bessere Diskriminierung zwischen full match- und mismatch-Bereichen entsteht.
In **Abbildung 4** ist eine Abreicherung unerwünschter Sequenzen aus der Probe dargestellt. Das Hybridisierungsverfahren erfolgt im Kreisfluss mit oder ohne fluktuierendem Temperaturprofil. Die Bedingungen dieses Verfahrens sind eine hohe lokale Probenkonzentration, das Bereitstellen längerer Rezeptoren für repetitive Sequenzen oder/und das Einstellen einer Temperatur über dem Schmelzpunkt eines Hybrids zwischen der Zielsequenz und den kürzeren Rezeptorsonden auf dem Träger, die an nichtrepetitive Sequenzen aus der Probe binden. Bei dem ersten Hybridisierungszyklus hybridisieren die repetitiven Sequenzen, die aufgrund ihrer höheren relativen Konzentration aus kinetischen Gründen schneller hybridisieren. Somit werden die repetitiven Sequenzen aus der Lösung abgereichert und die abgereicherte Lösung wird in einem Reservoir zwischengelagert. Anschließend kann die Temperatur in den Mikrokanälen erhöht werden, so dass es zur Dehybridisierung der repetitiven DNA-Moleküle kommt, die nun in ein weiteres Reservoir, z.B. ein Abfallreservoir, gespült werden können. Anschließend wird die abgereicherte Probenlösung bei niedrigerer Temperatur erneut hybridisiert. Der Prozess kann je nach Bedingungen und Probenzusammensetzungen auch mehrfach wiederholt werden. Alternativ kann man die abgereicherte Probenlösung auch in einen "frischen" Kanal umleiten.

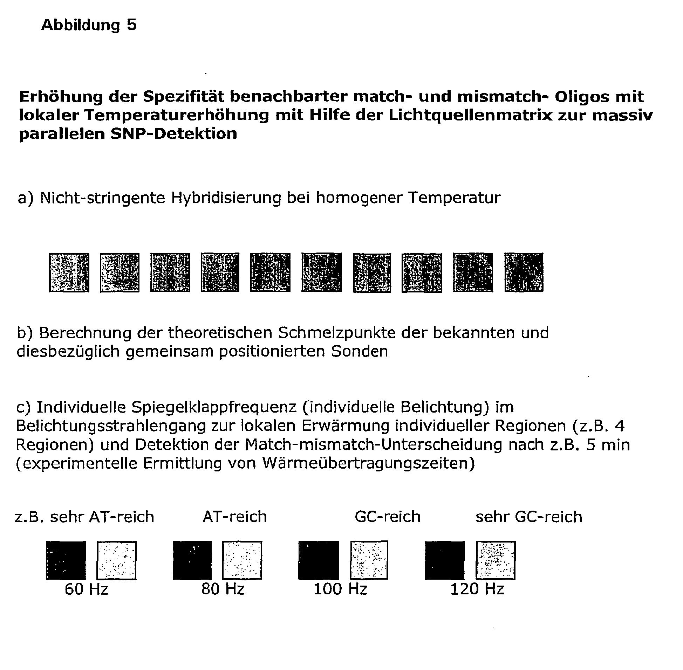

In **Abbildung 5** wird gezeigt wie die lokale Temperaturerhöhung mit Hilfe einer Lichtquellenmatrix die Spezifität benachbarter Match- und Mismatch-Rezeptoren erhöht und so eine massive parallele SNP-Detektion ermöglicht. Bei homogener Temperatur erfolgt eine nicht-stringente Hybridisierung. Wenn man die theoretischen Schmelzpunkte der bekannten Sonden berechnet, so kann man in den unterschiedlichen Bereichen individuelle Spiegelklappfrequenzen (individuelle Belichtung) im Belichtungsstrahlengang einstellen, um so eine lokale Erwärmung individueller Regionen zu erzeugen. Mit diesem Verfahren ist die Detektion der Match-mismatch-Unterscheidung möglich. Dieses Prinzip kann auch direkt bei der Hybridisierung benutzt werden. Hier bauen sich Temperaturgradienten auf, die eine gleichzeitige Hybridisierung vieler DNA-Stränge unterschiedlicher Schmelztemperatur ermöglichen.
**Abbildung 6** zeigt schematisch, wie nach einem Hybridisierungsverfahren die Detektion mit homogener Spiegelklappfrequenz erfolgt. Man kann gesättigte Bereiche erkennen, jedoch auch Bereiche, in denen das Signal im Hintergrundrauschen verloren geht und so nicht identifizierbar ist. Um eine Erhöhung der Sensitivität zu erhalten, muss die Einstrahlung von Anregungslicht, z.B. über die lokalen Spiegelklappfrequenzen, angepasst werden, wobei starke Signale proportional weniger häufig belichtet werden als schwache Signale. Es erfolgt eine Positionierung im linearen dynamischen Messbereich des Detektors, z.B. einer CCD-Kamera. Eventuell muss eine zweite Anpassung der lokalen Spiegelklappfrequenz erfolgen bis das Messsignal einheitlich ist und bis das Signal im optimalen Messbereich des Detektors liegt. Anschließend wird die Fluoreszenzintensität über die Spiegelklappfrequenz berechnet.

### Ausführungsbeispiele

### Beispiel 1: Expression Profiling mit komplettem Hefegenom (6000 Gene)

Es werden Träger hergestellt mit je 500 Positionen für GAPDH, Actin u.a. bekanntermaßen hochexprimierte Gene und mit je einer Position für alle anderen selten exprimierte Gene. Ein Hybridisierungsexperiment wird durchgeführt. Gemäß der gemessenen Signalintensitäten werden die individuellen Stellplätze und die individuellen Stellplatzdichten angepasst (Äquilibrierung der Schmelztemperaturen). Das Hybridisierungsverfahren wird wiederholt, wobei die Detektionszeiten, zur Erhöhung der Sensitivität im linearen Messbereich, verlängert werden. Die redundanten Stellplätze werden zu einem Messwert integriert.

### Beispiel 2: Sequenzierung mittels Hybridisierung (SbH) mit humaner BAC-Sequenz

Die Hälfte eines Trägers wird mit einem multifunktionalen Spacer und mit Rezeptorgemischen beladen, die zu den repetitiven Regionen und der Vektorsequenz komplementär sind. Diese Rezeptoren sind bis zu 50 Basen lang. Die andere Hälfte des Trägers wird mit kürzeren Rezeptoren zur Resequenzierung von Regionen aus Zielgenen beladen. Ein Temperaturgradient wird angelegt, wobei für repetitive Regionen, d.h. für lange Rezeptoren, höhere Temperaturen eingestellt werden, damit spezifische Sequenzen dort nicht durch Falschhybridisierung abgereichert werden, und für spezifische Regionen, d.h. kurze Rezeptoren, niedrige Temperaturen eingestellt werden. Das BAC-DNA wird statistisch fragmentiert und anschließend wird das Hybridisierungsverfahren durchgeführt. Eventuell wird die Hybridisierung zyklisch durchgeführt, um den Reassoziationskinetikeffekt auszunutzen. Die Signale werden im spezifischen Bereich mit verbessertem Signal-Hintergrundverhältnis detektiert.

### Beispiel 3

### Beispiel 3a: Sequenzierung mittels Hybridisierung (SbH) mit erhöhter Spezifität über einen fluktuierenden Temperaturgradienten

Dieses Beispiel ist schematisch in Abbildung 2 dargestellt. Es wird ein fluktuierender Temperaturgradient angelegt. Währenddessen erfolgt eine Hybridisierung im Kreislauf. Mit Hilfe der Fluidkonvektion in Kombination mit der fluktuierenden Temperierung wird ein thermodynamisches Gleichgewicht durch Lösen falschpositiver Hybridisierungsereignisse eingestellt (kinetisch bedingte lokale thermodynamische Minima).

### Beispiel 3b: Sequenzierung mittels Hybridisierung (SbH) mit erhöhter Spezifität über online-Beobachtung nach Hybridisierung und Temperaturerhöhung

Dieses Beispiel ist schematisch in Abbildung 3 dargestellt. Es werden Match- und Mismatch-Rezeptoren in direkter Nachbarschaft positioniert. Eine Hybridisierung mit einer Zielnukleinsäure in einem fluktuierenden Temperaturgradienten wird durchführt. Die Hybridisierungstemperatur wird langsam erhöht, währenddessen eine Messung des Hybridisierungssignals erfolgt. Die Detektion erfolgt mit online- oder Intervall-Beobachtung durch eine online-CCD-Kamera.

### Beispiel 3c: Sequenzierung mittels Hybridisierung (SbH) mit erhöhter Spezifität über lokale Temperaturführung vor oder/und nach Hybridisierung

Dieses Beispiel ist schematisch in Abbildung 5 dargestellt. Es werden Match- und Mismatch-Rezeptoren in direkter Nachbarschaft auf dem Träger positioniert. Die Hybridisierung erfolgt mit einer Zielnukleinsäure in einem fluktuierenden Temperaturgradienten. Die Hybridisierungstemperatur wird eingestellt und anschließend werden lokal unterschiedliche Wärmemengen mit Hilfe lokaler Belichtung durch eine Lichtquellenmatrix eingebracht. Hierzu werden vorzugsweise IR-Strahlungen als Lichtquelle benutzt. Die Ermittlung einer Intensitätsdifferenz individueller Rezeptor-Paare zu einem beliebigen Zeitpunkt führt zu einer Einzelbasen-Match-Mismatch-Unterscheidung.

## Patentansprüche

1. Verfahren zur Bestimmung von Nukleinsäureanalyten durch Hybridisierung, umfassend die Schritte
(a) Bereitstellen eines mikrofluidischen Trägers mit Kanälen,
(b) Synthetisieren von jeweils unterschiedlichen Rezeptoren in situ an mehreren vorbestimmten Bereichen auf dem mikrofluidischen Träger, wobei die Rezeptoren an den mehreren vorbestimmten Bereichen auf dem Träger immobilisiert werden und sich die mehreren vorbestimmten Bereiche mit immobilisierten Rezeptoren in den Kanälen des mikrofluidischen Trägers befinden,
(c) Inkontaktbringen des mikrofluidischen Trägers mit einer Analyten enthaltenden Probe und
(d) Bestimmen der Analyten über deren Bindung an die auf dem mikrofluidischen Träger immobilisierten Rezeptoren,
wobei für vorbestimmte Bereiche oder Gruppen von Bereichen mit Rezeptoren jeweils eine unterschiedliche Intensität des Messsignals durch lokal unterschiedliche Einstrahlung von Anregungslicht zur Anpassung der Fluoreszenzintensität in den vorbestimmten Bereichen erzeugt wird, so dass der lineare dynamische Messbereich des Detektors nicht überschritten wird, und der Nachweis des Analyten durch Fluoreszenz erfolgt, und wobei die Rezeptoren Hybridisierungssonden sind.

2. Verfahren nach Anspruch 1,
**dadurch gekennzeichnet,**
**dass** die lokal unterschiedliche Einstrahlung von Anregungslicht über eine Lichtquellenmatrix erfolgt.

3. Verfahren nach Anspruch 1,
**dadurch gekennzeichnet,**
**dass** die Belichtungsintensitäten in einzelnen Bereichen um mindestens 50 %, vorzugsweise um mindestens 100 % variiere, basierend auf dem Bereich mit der geringsten Beleuchtungsintensität.

4. Verfahren nach einem der Ansprüche 1 bis 3,
**dadurch gekennzeichnet,**
**dass** man mehrere Analyten, vorzugsweise mindestens 50 Analyten und besonders bevorzugt mindestens mindestens 100 Analyten, parallel in der Probe bestimmt.

5. Verfahren nach einem der Ansprüche 1 bis 4,
**dadurch gekennzeichnet,**
**dass** die Rezeptoren aus Nukleinsäuren und Nukleinsäureanaloga ausgewählt werden und die Bindung der Analyten an die Rezeptoren eine Hybridisierung umfasst.

6. Verfahren nach einem der Ansprüche 1 bis 5,
**dadurch gekennzeichnet,**
**dass** zur Bestimmung der Analyten eine Vorrichtung verwendet wird, umfassend
(i) eine Lichtquellenmatrix,
(ii) den mikrofluidischen Träger,
(iii) ein Mittel zur Zufuhr von Fluid zum Träger und zur Ableitung von Fluids aus dem Träger und
(iv) eine Detektionsmatrix.

7. Verfahren nach Anspruch 6,
**dadurch gekennzeichnet,**
**dass** eine programmierbare Lichtquellenmatrix, ausgewählt aus einer Lichtventilmatrix, einem Spiegelarray und einem UV-Laser-Array, verwendet wird.

8. Verfahren nach Anspruch 7,
**dadurch gekennzeichnet,**
**dass** eine programmierbare Detektionsmatrix, ausgewählt aus einem CCD-Array, lichtsensitiven Halbleiterstrukturen und elektronischen Detektoren, verwendet wird.

9. Verfahren nach einem der Ansprüche 1 bis 8,
**dadurch gekennzeichnet,**
**dass** die Synthese der Rezeptoren umfasst:
das Leiten von Fluid mit Rezeptor-Synthesebausteinen über den Träger, das orts- oder/und zeitspezifische Immobilisieren der Bausteine an den jeweils vorbestimmten Bereichen auf dem Träger und das Wiederholen dieser Schritte, bis die gewünschten Rezeptoren an den jeweils vorbestimmten Bereichen synthetisiert worden sind.

10. Verfahren nach Anspruch 9,
**dadurch gekennzeichnet,**
**dass** die Synthese der Rezeptoren fluidchemische Reaktionsschritte oder/und Belichtungsschritte oder/und elektrochemische Reaktionsschritte umfasst.

## Claims

1. A method for determining nucleic acid analytes by hybridisation, comprising the steps of
(a) preparing a microfluid support with channels,
(b) synthesing different receptors respectively in situ at a plurality of predetermined zones on the microfluid support,
wherein the receptors are immobilised at the plurality of predetermined zones on the support and the plurality of predetermined zones having immobilised receptors are situated in the channels of the microfluid support,
(c) contacting the microfluid support with a sample containing analytes and
(d) determining the analytes via their binding to the receptors immobilised on the microfluid support,
wherein for predetermined zones or groups of zones having receptors a different intensity of the measurement signal is induced in each case by locally varying the irradiation by excitation light to adjust the fluorescence intensity in the predetermined zones so that the linear dynamic measurement range of the detector is not exceeded, and the detection of the analytes is carried out by fluorescence, and wherein the receptors are hybridisation probes.

2. A method according to Claim 1,
**characterised in that** the locally varying irradiation of excitation light is effected via a light source matrix.

3. A method according to Claim 1,
**characterised in that** the exposure intensities in individual zones vary by at least 50 %, preferably by at least 100 %, based on the zone with the lowest exposure intensity.

4. A method according to any one of Claims 1 to 3,
**characterised in that** a plurality of analytes are determined in parallel in the sample, preferably at least 50 analytes and especially preferably at least 100 analatyes.

5. A method according to any one of Claims 1 to 4,
**characterised in that** the receptors are selected from nucleic acids and nucleic acid analogues and the binding of the analytes to the receptors comprises a hybridisation.

6. A method according to any of one of Claims 1 to 5,
**characterised in that** an apparatus is used to determine the analytes, comprising
(i) a light source matrix,
(ii) the microfluid support,
(iii) a means for supplying fluid to the support and for discharging fluid from the support and
(iv) a detection matrix.

7. A method according to Claim 6,
**characterised in that** a programmable light source matrix, selected from a light valve matrix, a mirror array and a UV laser array, is used.

8. A method according to Claim 7,
**characterised in that** a programmable detection matrix, selected from a CCD array, light-sensitive semiconductor structures and electronic detectors, is used.

9. A method according to any one of Claims 1 to 8,
**characterised in that** the synthesis of the receptors comprises: the direction of fluid with receptor·synthesis components over the support, the location-specific and/or time-specific immobilisation of the components at the respective predetermined zones on the support and the repetition of these steps until the desired receptors have been synthesized at the respective predetermined zones.

10. A method according to Claim 9,
**characterised in that** synthesis of the receptors comprises fluid-chemical reaction steps and/or exposure steps and/or electrochemical reaction steps.

## Revendications

1. Procédé de détermination d'analytes d'acides nucléiques par hybridation, comprenant les étapes consistant à :
(a) mettre à disposition un support microfluidique comportant des canaux,
(b) synthétiser des récepteurs respectivement différents in situ à plusieurs endroits prédéterminés sur le support microfluidique, les récepteurs étant immobilisés sur le support à plusieurs endroits prédéterminés et les multiples endroits prédéterminés avec les récepteurs immobilisés se trouvant dans les canaux du support microfluidique,
(c) mettre en contact le support microfluidique avec un échantillon contenant des analytes et
(d) déterminer les analytes par le biais de leur liaison aux récepteurs immobilisés sur le support microfluidique,
dans lequel, pour des endroits ou groupes d'endroits prédéterminés comportant des récepteurs, une intensité du signal de mesure différente est à chaque fois produite par irradiation d'une lumière d'excitation localement différente afin d'ajuster l'intensité de fluorescence dans les endroits prédéterminés pour que l'intervalle de mesure dynamique linéaire du détecteur ne soit pas dépassé, et la détection de l'analyte s'effectue par fluorescence et
dans lequel les récepteurs sont des sondes d'hybridation.

2. Procédé selon la revendication 1, **caractérisé en ce que** l'irradiation de la lumière d'excitation différente localement s'effectue par le biais d'une matrice de sources lumineuses.

3. Procédé selon la revendication 1, **caractérisé en ce que** les intensités d'illumination varient dans les différents endroits d'au moins 50 %, de préférence d'au moins 100 %, sur la base de l'endroit ayant l'intensité d'illumination la plus faible.

4. Procédé selon l'une des revendications 1 à 3, **caractérisé en ce que** l'on détermine plusieurs analytes, de préférence au moins 50 analytes et particulièrement préférentiellement au moins 100 analytes, en parallèle dans l'échantillon.

5. Procédé selon l'une des revendications 1 à 4, **caractérisé en ce que** les récepteurs sont choisis parmi les acides nucléiques et les analogues d'acides nucléiques et la liaison des analytes aux récepteurs comprend une hybridation.

6. Procédé selon l'une des revendications 1 à 5, **caractérisé en ce que**, pour déterminer les analytes, on utilise un dispositif comprenant :
(i) une matrice de sources lumineuses,
(ii) le support microfluidique,
(iii) un agent pour l'apport de fluide au support et pour l'évacuation du fluide hors du support et
(iv) une matrice de détection.

7. Procédé selon la revendication 6, **caractérisé en ce que** l'on utilise une matrice de sources lumineuses programmable, choisie parmi une matrice à clapet lumineux, un réseau de miroirs, et un réseau de lasers UV.

8. Procédé selon la revendication 7, **caractérisé en ce que** l'on utilise une matrice de détection programmable, choisie parmi un réseau CCD, des structures semi-conductrices photosensibles et des détecteurs électroniques.

9. Procédé selon l'une des revendications 1 à 8, **caractérisé en ce que** la synthèse des récepteurs comprend :
la conduction du fluide avec des composants de synthèse des récepteurs au-dessus du support, l'immobilisation des composants, spécifique à un endroit et/ou à un moment, sur le support à des endroits respectivement prédéterminés et la répétition de ces étapes, jusqu'à ce que les récepteurs voulus aient été synthétisés aux endroits respectivement prédéterminés.

10. Procédé selon revendication 9, **caractérisé en ce que** la synthèse des récepteurs comprend des étapes de réaction de la chimie des fluides et/ou des étapes d'illumination et/ou des étapes de réaction électrochimique.
